# FASCICULE DE BREVET EUROPEEN

(11) **EP 2 958 650 B1**
(45) Date de publication et mention de la délivrance du brevet: **20.07.2022**
(21) Numéro de dépôt: 14705172.6
(22) Date de dépôt: 18.02.2014
(51) Int. Cl.: B01D 15/18, B01J 20/18, C07C 7/13, C07C 15/08, B01J 20/28

(54) **PROCÉDÉ DE SÉPARATION DES XYLÈNES EN LIT MOBILE SIMULÉ AU MOYEN D'UN SOLIDE ADSORBANT ZÉOLITHIQUE DE GRANULOMÉTRIE COMPRISE ENTRE 150 ET 500 MICRONS**
VERFAHREN ZUR TRENNUNG VON XYLOLEN IN EINEM SIMULIERTEN WANDERBETT MITTELS EINES ZEOLITHISCHEN ADSORBIERENDEN FESTSTOFFES MIT EINER TEILCHENGRÖSSE ZWISCHEN 150 UND 500 MIKRON
METHOD FOR SEPARATING XYLENES IN A SIMULATED MOVING BED BY MEANS OF A ZEOLITIC ADSORBENT SOLID HAVING A PARTICLE SIZE OF BETWEEN 150 AND 500 MICRONS

(30) Priorité: 22.02.2013 FR 1351536
(43) Date de publication de la demande: 30.12.2015
(73) Titulaire: IFP Energies nouvelles, 92500 Rueil-Malmaison (FR); ARKEMA FRANCE, 92700 Colombes (FR)
(72) Inventeur: LAROCHE, Catherine, 69390 Vernaison (FR); LEINEKUGEL LE COCQ, Damien, 69600 Oullins (FR); LEFLAIVE, Philibert, 69780 Mions (FR); AUGIER, Frédéric, 69360 Saint-Symphorien-D'Ozon (FR); BOUVIER, Ludivine, 64300 Orthez (FR); LUTZ, Cécile, 64290 Gan (FR); SZENDROVICS, Sylvie, 64370 Arthez-de-Béarn (FR); PERSILLON, Quitterie, 64160 Morlaas (FR)
(74) Mandataire: Bandpay & Greuter
(86) Numéro de dépôt international: PCT/EP2014/053138
(87) Numéro de publication internationale: WO 2014/128125

(56) Documents cités:
- FR-A1- 2 693 186
- FR-A1- 2 795 407
- FR-A1- 2 903 978
- FR-A1- 2 925 366
- US-A1- 2009 326 309
- US-A1- 2011 105 301

## Description

### DOMAINE DE L'INVENTION

La présente invention concerne le domaine des procédés de séparation des xylènes en lit mobile simulé (noté en abrégé LMS).

L'invention a pour but d'améliorer la productivité à long terme des procédés existants en jouant sur les caractéristiques de granulométrie des particules de solide adsorbant.

La séparation en lit mobile simulé s'entend ici au sens large, c'est-à-dire qu'on peut avoir affaire soit à un contre-courant simulé, soit à un co-courant simulé, soit à un procédé dit "varicol".

La caractéristique commune à cette famille de procédés est que le solide adsorbant est mis en œuvre en lit fixe, et que les flux liquides en contact avec l'adsorbant sont gérés soit au moyen d'un jeu de vannes tout ou rien, soit au moyen d'une vanne unique complexe connue sous le nom de vanne rotative ou "rotary valve".

Lorsque l'élément actif des solides adsorbants utilisés comme agents d'adsorption dans ces procédés est une zéolithe, cette dernière, obtenue sous forme de poudre, est de préférence employée à l'échelle industrielle sous forme d'agglomérés. Ces adsorbants zéolithiques, agglomérés sous forme de plaquettes, billes ou extrudés, sont en général constitués d'une poudre de zéolithe, qui constitue l'élément actif au sens de l'adsorption et d'un liant destiné à assurer la cohésion des cristaux sous forme de grains. Ce liant confère également aux grains une résistance mécanique suffisante pour résister aux contraintes mécaniques auxquels ils sont soumis au cours de leur mise en œuvre au sein des unités. Ces contraintes mécaniques sont à l'origine de la formation de fines, qui induisent une détérioration des performances au cours de la durée d'opération du procédé.

Le procédé de séparation des xylènes en lit mobile simulé (LMS) a connu de très nombreuses améliorations technologiques, notamment au niveau des plateaux distributeurs des fluides, mais relativement peu d'évolution concernant les caractéristiques granulométriques des particules du solide adsorbant.

### ARRIERE-PLAN TECHNOLOGIQUE

Les propriétés recherchées au niveau du solide adsorbant zéolithique sont les suivantes :
- Capacité d'adsorption de l'aggloméré zéolithique la plus grande possible, c'est à dire teneur en zéolithe la plus grande possible, la zéolithe constituant la microporosité au sein de laquelle a lieu l'adsorption.
- Transfert au sein de l'aggloméré zéolithique le plus fort possible, c'est à dire temps minimal pour une molécule d'hydrocarbure pour aller de l'extérieur de l'aggloméré au cœur des cristaux de zéolithe de l'adsorbant zéolithique.
- Résistance mécanique à l'écrasement de l'aggloméré zéolithique la plus forte possible, et en tous cas supérieure à 1,7 MPa telle que mesurée par le test Shell de résistance à l'écrasement en lit fixe, modifié pour s'adapter à la granulométrie des agglomérés.

Les documents de l'art antérieur décrivant les caractéristiques chimiques et microscopiques des adsorbants zéolithiques utilisés pour la séparation du paraxylène sont particulièrement nombreux par exemple (US 3,558,730 ; US 3,663,638 (Neuzil) ; US 3,960,774 (Rosback) ; US 6,706,938 (Roeseler) ; US 7,820,869 (Priegnitz) ; US 7,812,208 (Cheng) ; US 6,410,815 ; US 6,884,918 (Plee) ; WO08/009845 ; WO09/081023 (Bouvier) ; US2011/105301 (Wang)). L'enseignement général sur les caractéristiques chimiques de ces solides adsorbants est qu'il faut utiliser une zéolithe de structure faujasite (zéolithe LSX, X ou Y) échangée au baryum (à au moins 90%, exprimé en taux d'échange) ou échangée très majoritairement au baryum et de manière minoritaire au potassium (par exemple de 2 à 33%). Pour la séparation du métaxylène, US 5,382,747 (Kulprathipanja) enseigne l'utilisation d'une zéolithe Y au sodium ou d'une zéolithe Y au sodium partiellement échangée au lithium.

L'enseignement général sur les caractéristiques microscopiques de l'adsorbant est que l'on peut utiliser des cristaux de zéolithe LSX de taille de préférence inférieure à 2 microns (diamètre moyen en nombre) ou des cristaux de zéolithe X de taille de préférence inférieure à 1,6 microns (diamètre moyen en nombre). La taille des cristaux de zéolithe Y conventionnelle est de l'ordre de 1 à 3 µm, et US 7,728,187 (Kulprathipanja) préconise l'utilisation de nano-cristaux de zéolithe Y, de taille comprise entre 50 et 700 nm, pour améliorer le transfert de matière du métaxylène. Une caractéristique supplémentaire de ces zéolithes est qu'elles doivent être hydratées de manière à présenter un transfert microporeux acceptable (US 5,107,062 (Zinnen)).

L'obtention d'un transfert microporeux le plus fort possible est alors en contradiction avec l'obtention d'une capacité d'adsorption la plus forte possible, puisque la présence d'eau dans la structure zéolithique va réduire la capacité totale d'adsorption des xylènes ainsi que la capacité d'adsorption sélective du para-xylène (US 7,820,869 (Priegnitz) ou du méta-xylène (US 5,382,747 (Kulprathipanja)).

Par conséquent, il faut parfois ajuster l'hydratation de la zéolithe pour réaliser un compromis entre un transfert microporeux et une capacité d'adsorption sélective acceptables. L'ajustement du taux d'hydratation de l'adsorbant peut être réalisé en variant les conditions d'activation finale de l'adsorbant (c'est à dire température, durée...), qui constitue la dernière étape de la fabrication de l'adsorbant.

La mesure de l'hydratation de l'adsorbant peut être évaluée par approximation avec la mesure de la perte au feu, qui peut être réalisée sur l'adsorbant avant qu'il soit introduit dans l'unité et mis au contact avec les xylènes. Typiquement une zéolithe LSX ou une zéolithe X doivent présenter une perte au feu à 900°C comprise entre 4 et 7,7 % et une zéolithe Y doit présenter une perte au feu à 900°C comprise entre 0 et 3%.

L'enseignement général concernant les caractéristiques macroscopiques de l'adsorbant est que l'on peut augmenter la teneur en matière active, par transformation du liant en zéolithe sous l'action d'une solution alcaline basique, de sorte que le produit fini contienne une quantité de phase non zéolithique réduite, qui peut être quantifiée par référence à un adsorbant composé uniquement de zéolithe, sous forme de poudre, à partir de mesures d'adsorption ou à partir d'intensités de pics de DRX. Cette transformation du liant en matière active au sens de l'adsorption permet par ailleurs de maintenir la résistance mécanique de l'aggloméré (WO08/009845 (Bouvier)), ce qui est nécessaire pour résister aux contraintes mécaniques lors de leur mise en œuvre au sein des unités.

L'autre enseignement général sur les caractéristiques macroscopiques de l'adsorbant est que les agglomérés sont en général de granulométrie comprise entre 0,2 mm et 1,5 mm et plus particulièrement entre 0,25 et 1,2 mm (16-60 Standard US Mesh size - US 7,820,869 (Priegnitz)) ou entre 0,35 et 0,8mm (Ex. 1 US2011/105301 (Wang)). Cette gamme granulométrique est obtenue par tamisage et/ou cyclonage à l'issue de l'étape de mise en forme des agglomérés. Les documents cités précédemment fournissent des indications sur le "size range", c'est à dire la gamme granulométrique des agglomérés et non sur le diamètre moyen de ces agglomérés. WO08/009845 et WO09/081023 (Bouvier) enseignent que les agglomérés ont en général un diamètre moyen en nombre allant de 0,4 à 2 mm et en particulier de 0,4 à 0,8 mm.

Dans le procédé de séparation des xylènes par adsorption en lit mobile simulé, le solide adsorbant zéolithique est mis en contact avec le flux liquide d'alimentation (charge) composé du mélange de xylènes, contenant ortho-xylène, méta-xylène, para-xylène et ethylbenzène.

En utilisant un adsorbant zéolithique à base de zéolithe de structure faujasite de rapport Si/Al compris entre 1 et 1,5 (zéolithe LSX, X) échangée au baryum (à au moins 90% exprimé en taux d'échange) ou échangée très majoritairement au baryum et de manière minoritaire au potassium, le para-xylène est alors adsorbé dans les micropores de la zéolithe préférentiellement par rapport à l'ensemble des autres composés hydrocarbures présents dans le flux d'alimentation. La phase adsorbée dans les micropores de la zéolithe se trouve alors enrichie en para-xylène par rapport au mélange initial constituant le flux d'alimentation. La phase liquide se trouve à l'inverse enrichie en composés tels que ortho-xylène, méta-xylène et ethylbenzène en proportion relative plus importante que celle caractérisant le mélange initial constituant le flux d'alimentation.

En utilisant un adsorbant zéolithique à base de zéolithe de structure faujasite de rapport Si/Al compris entre 1,5 < Si/Al < 6 (zéolithe Y) échangée au sodium ou échangée au sodium et lithium, le méta-xylène est alors adsorbé dans les micropores de la zéolithe préférentiellement par rapport à l'ensemble des autres composés hydrocarbures présents dans le flux d'alimentation. La phase adsorbée dans les micropores de la zéolithe se trouve alors enrichie en méta-xylène par rapport au mélange initial constituant le flux d'alimentation. La phase liquide se trouve à l'inverse enrichie en composés tels que ortho-xylène, para-xylène et ethylbenzène en proportion relative plus importante que celle caractérisant le mélange initial constituant le flux d'alimentation.

La phase liquide est soustraite du contact avec l'adsorbant formant ainsi un flux de raffinat.

La phase adsorbée, enrichie en para-xylène ou méta-xylène selon la zéolithe utilisée dans l'adsorbant, est désorbée sous l'action d'un flux de désorbant, et soustraite du contact avec l'adsorbant formant alors un flux d'extrait.

Le maintien de l'hydratation de la zéolithe à la valeur souhaitée, par exemple une perte au feu de 4% à 7,7% pour la zéolithe X ou LSX et 0 à 3% pour la zéolithe Y, lors de sa mise en œuvre dans le procédé de séparation des xylènes par adsorption en lit mobile simulé, est assuré en ajoutant de l'eau dans les flux d'alimentation de la charge et/ou de désorbant. L'addition d'eau nécessaire pour de tels niveaux de perte au feu est telle que la teneur en eau en poids dans les effluents hydrocarbonés (le flux d'extrait ou de raffinat) est comprise entre 0 ppm et 150 ppm (40-150 ppm lorsque l'adsorbant est à base de zéolithe X ou LSX et 0-80ppm lorsque l'adsorbant est à base de zéolithe Y).

Dans le procédé de séparation des xylènes par adsorption en lit mobile simulé, le solide adsorbant zéolithique est mis en œuvre dans une ou deux colonnes multi étagées pour être mis en contact avec le flux de liquide. On appelle colonne multi étagée une colonne constituée d'une multiplicité de plateaux disposés selon un axe sensiblement vertical, chaque plateau supportant un lit de solide granulaire, et les différents lits successifs étant traversés en série par le ou les fluides mis en œuvre dans la colonne. Entre deux lits successifs est situé un dispositif de distribution des fluides permettant d'alimenter chaque lit de solide granulaire.

Le lit de solide granulaire peut être bloqué par le dispositif de distribution, mais le plus souvent, il existe un espace vide entre le dispositif de distribution et la surface du lit de solide granulaire aval afin de permettre un léger fléchissement du dispositif de distribution.

De manière générale le fonctionnement d'une colonne en lit mobile simulé peut être décrit de la façon suivante:
Une colonne comporte au moins quatre zones, et éventuellement cinq ou six, chacune de ces zones étant constituée par un certain nombre de lits successifs, et chaque zone étant définie par sa position comprise entre un point d'alimentation et un point de soutirage. Typiquement, une unité CCS pour la production de paraxylène ou la production de métaxylène est alimentée par au moins une charge F à fractionner (charge d'hydrocarbures aromatiques constituée des isomères à 8 atomes de carbones) et un désorbant D, parfois appelé éluant (généralement du paradiéthyl benzène ou du toluène), et l'on soutire de ladite unité au moins un raffinat R contenant les produits de la charge les moins sélectivement adsorbés et du désorbant et un extrait E contenant le produit de la charge le plus adsorbé et du désorbant.

D'autres points d'injection et de soutirage peuvent être ajoutés de manière à rincer les circuits de distribution, comme décrit par exemple dans le brevet US 7,208,651. L'ajout de ces flux supplémentaires de rinçage ne changeant en rien le principe de fonctionnement du CCS, dans un souci de concision, nous n'ajouterons pas ces points d'injection et de soutirage supplémentaires dans la description du procédé selon l'invention.

Les points d'alimentation et de soutirage sont modifiés au cours du temps, décalés dans le même sens d'une valeur correspondant à un lit. Les décalages des différents points d'injection ou de soutirage peuvent être soit simultanés, soit nonsimultanés comme l'enseigne le brevet US 6,136,198. Le procédé selon ce second mode de fonctionnement est appelé VARICOL.

Classiquement, on définit 4 zones chromatographiques différentes dans une colonne fonctionnant en contre-courant simulé (CCS).
- Zone 1 : zone de désorption du produit de la charge le plus adsorbé, comprise entre l'injection du désorbant D et le prélèvement de l'extrait E.
- Zone 2 : zone de désorption des produits de la charge les moins sélectivement adsorbés, comprise entre le prélèvement de l'extrait E et l'injection de la charge à fractionner F.
- Zone 3 : zone d'adsorption du produit de la charge le plus adsorbé, comprise entre l'injection de la charge et le soutirage du raffinat R.
- Zone 4 : zone située entre le soutirage de raffinat R et l'injection du désorbant D.

Pour augmenter la productivité du procédé de séparation, on cherchera à obtenir des adsorbants zéolithiques du plus petit diamètre possible afin d'augmenter le transfert.

Les documents de l'art antérieur décrivant des procédés de séparation pharmaceutique (Gomes et al., 2006, Adsorption, vol.12, p.375) rapportent des procédés de séparation chromatographique en phase liquide utilisant des agglomérés de quelques dizaines de micromètres à 100µm.

Dans ces procédés utilisant des agglomérés de très petite taille, la perte de charge ΔP est alors élevée. Dans les procédés de séparation des xylènes, de tels niveaux de perte de charge ΔP ne sont pas courants. On remarque néanmoins de façon étonnante que la perte de charge ΔP n'est pas un critère dimensionnant. Il aura un impact notamment sur l'épaisseur des parois de l'adsorbeur et sur la puissance des installations.

Dans le procédé de séparation des xylènes par adsorption en lit mobile simulé, l'adsorbant zéolithique est supporté par des plateaux formant des lits de solide granulaire successifs traversés en série par le ou les fluides mis en œuvre dans la colonne. Entre deux lits successifs est situé un dispositif de distribution des fluides permettant d'alimenter chaque lit de solide granulaire et le plus souvent, il existe un espace vide entre le dispositif de distribution et la surface du lit de solide granulaire aval.

Les plateaux peuvent avoir une découpe en panneaux de type parallèles (panneaux méridiens) ou de type radiaux (panneaux en parts de tartes ou pie-cut en anglais). Chaque plateau est délimité par une grille inférieure et une grille supérieure. Un volume ne contenant pas d'adsorbant est situé entre la grille inférieure d'un plateau et le haut du lit placé en-dessous du plateau considéré. Ce volume est nécessaire pour éviter tout phénomène de dégradation mécanique du solide adsorbant lié au fléchissement du plateau.

De façon inhérente à la distribution, il existe une composante tangentielle du flux qui favorise le phénomène de fluidisation partielle ou d'entraînement des particules à la surface du lit de solide granulaire, provoquant la formation de sillons ou talus à la surface du lit. Un tel phénomène est favorisé par les vitesses de circulation élevées et les diamètres de particules faibles.

La formation de talus a deux effets néfastes sur le fonctionnement de l'unité. D'une part, cela perturbe le flux et génère des retards de temps de séjour dégradant les performances de la séparation. D'autre part, cela favorise l'écrasement du tamis par les plateaux ou les supports de plateaux. Or la durée de vie du tamis est en majeure partie liée à la génération de fines par écrasement du tamis.

Il a été découvert que le choix d'une gamme particulière de taille des billes pour un adsorbant zéolithique aggloméré permettait de s'affranchir de tels phénomènes, et d'augmenter ainsi la durée de vie du tamis.

La présente invention décrit un procédé mettant en œuvre un adsorbant aggloméré dont la granulométrie particulière est choisie de façon à éviter la dégradation des performances du procédé à long terme. Le choix de la granulométrie particulière permet d'éviter la formation de talus à la surface des lits de solide granulaire dans la ou les colonne(s) du lit mobile simulé.

La présente invention propose ainsi un procédé de séparation des xylènes mettant en œuvre un adsorbant aggloméré de granulométrie réduite, préférentiellement à base de zéolithe faujasite de rapport Si/Al compris entre 1 et 6, permettant la production de paraxylène ou de métaxylène à haute pureté avec une productivité améliorée de façon durable, tout en évitant la dégradation des performances au cours du temps.

### DESCRIPTION DE L'INVENTION

### Résumé de l'invention

L'invention concerne un procédé de séparation des xylènes à partir d'une charge comprenant des coupes d'isomères d'hydrocarbures aromatiques contenant 8 atomes de carbone, en lit mobile simulé, par adsorption sélective d'un isomère du xylène en présence d'un désorbant, au moyen de particules d'adsorbant zéolithique aggloméré, sous forme de billes, à base de cristaux de zéolithe de diamètre moyen en nombre inférieur ou égal à 1,2 µm, dans lequel le diamètre moyen en nombre desdites particules d'adsorbant est compris entre 150 µm et 500 µm et la résistance mécanique mesurée par la méthode Shell série SMS1471-74 adaptée pour les agglomérés de taille inférieure à 500 µm, c'est-à-dire utilisant un échantillon tamisé avec un tamis de 100µm et séché à une température de 250°C, est supérieure ou égale à 2 MPa, le temps de cycle, correspondant au temps entre deux injections de désorbant sur un lit donné, étant compris entre 4 et 18 min et le rapport des débits de désorbant sur charge étant compris entre 0,7 et 2,5 et le taux de recyclage étant compris entre 2,0 et 12.

Le diamètre moyen en nombre desdites particules d'adsorbant est de préférence compris entre 200 µm et 400 µm.

De préférence, la répartition granulométrique desdites particules d'adsorbant est telle qu'il n'existe aucune particule de taille inférieure à 100µm.

De manière préférée, le diamètre moyen en nombre des cristaux de zéolithe est compris entre 0,1 µm et 1,2 µm.

De manière très préférée, le diamètre moyen en nombre des cristaux de zéolithe est compris entre 0,5 µm et 0,8 µm.

Tout intervalle de valeurs désigné par l'expression "entre a et b" représente le domaine de valeurs allant de plus de a à moins de b (c'est-à-dire bornes a et b exclues), tandis que tout intervalle de valeurs désigné par l'expression "de a à b" signifie le domaine de valeurs allant de a jusqu'à b (c'est-à-dire incluant les bornes a et b).

Le procédé de séparation des xylènes peut être mis en œuvre dans une unité en lit mobile simulé ayant les caractéristiques suivantes:
- nombre de lits compris entre 4 et 24
- nombre de zones : au moins 4.

De manière avantageuse, le procédé de séparation des xylènes opère à une température de 100°C à 250°C, de préférence 120°C à 190°C et à une pression comprise entre la pression de bulle des xylènes à la température du procédé et 3 MPa.

De manière avantageuse, le rapport des débits de désorbant sur charge est compris entre 0,7 et 2,5 et le taux de recyclage est de 2,5 à 4.

Dans un mode de réalisation, l'invention concerne également un procédé de séparation du paraxylène par adsorption sélective du paraxylène dans lequel l'adsorbant zéolithique aggloméré est à base de zéolithe X ou LSX ayant un rapport atomique Si/Al tel que 1,0 ≤ Si/Al < 1,5, de préférence entre 1,1 ≤ Si/Al < 1,5, de préférence encore tel que 1,2 ≤ Si/Al < 1,3.

Dans ce cas, l'adsorbant zéolithique aggloméré peut comprendre en outre :
- Une teneur en oxyde de baryum BaO et une teneur en oxyde de potassium K₂O telle que le rapport entre le nombre de moles de l'ensemble oxyde de baryum + oxyde de potassium (BaO+K₂O) et le nombre de moles de l'ensemble (BaO+K₂O+Na₂O) est supérieur à 90% ;
- Une teneur en oxyde de potassium K₂O telle que le rapport entre le nombre de mole d'oxyde de potassium K₂O et le nombre de mole d'oxyde de baryum BaO est inférieur à 0,5 ;
- et une teneur totale en oxydes d'ions alcalins ou alcalino-terreux autres que le baryum et le potassium de préférence inférieure à 5% et de préférence allant de 0 à 2% et avantageusement allant de 0 à 1% en poids par rapport au poids total de l'adsorbant zéolithique.

Dans ce cas, l'adsorbant zéolithique aggloméré peut avoir une densité de grain comprise entre 1,1 et 1,4 g/mL, et de manière préférée entre 1,1 et 1,3 g/mL telle que mesurée par intrusion de mercure (exprimé par rapport à la masse sèche de l'adsorbant zéolithique) et un volume poreux total mesuré par intrusion de mercure (volume poreux contenu dans les macropores et les mésopores de diamètre apparent supérieur à 4 nm) compris entre 0,20 et 0,35 mL/g (exprimé par rapport à la masse sèche de l'adsorbant zéolithique).

Le procédé de séparation du paraxylène peut être mis en œuvre à une température de 165°C à 185°C et la teneur en eau dans les effluents hydrocarbonés est ajustée entre 20 ppm et 150 ppm, en ajoutant de l'eau dans la charge comprenant les coupes d'isomères d'hydrocarbures aromatiques contenant 8 atomes de carbone et/ou dans le désorbant.

Dans ledit procédé de séparation du paraxylène le désorbant est de préférence choisi parmi le toluène et le paradiéthylbenzène.

Dans un autre mode de réalisation, l'invention concerne un procédé de séparation du métaxylène par adsorption sélective du métaxylène, dans lequel l'adsorbant zéolithique aggloméré est à base de zéolithe Y ayant un rapport atomique Si/Al tel que 1,5 < Si/Al < 6, de préférence tel que 2,5 < Si/Al < 3.

Dans ce cas, l'adsorbant zéolithique aggloméré peut comprendre en outre :
- Une teneur en oxyde de sodium Na₂O et une teneur en oxyde de lithium Li₂O telle que le rapport entre le nombre de moles d'oxyde de sodium et le nombre de moles de l'ensemble oxyde de sodium + oxyde de lithium (Na₂O+Li₂O) est supérieur à 65%.
- Et une teneur totale en oxydes d'ions alcalins ou alcalino-terreux autres que le sodium et le lithium de préférence inférieure à 5% et de préférence allant de 0 à 2% et avantageusement allant de 0 à 1% en poids par rapport au poids total de l'adsorbant zéolithique.

Le procédé de séparation du métaxylène peut être mis en œuvre à une température de 120°C à 180°C et la teneur en eau dans les effluents hydrocarbonés est ajustée entre 0 ppm et 80 ppm, en ajoutant de l'eau dans la charge comprenant les coupes d'isomères d'hydrocarbures aromatiques contenant 8 atomes de carbone et/ou dans le désorbant.

Dans le procédé de séparation du métaxylène selon l'invention, le désorbant est de préférence choisi parmi le toluène et l'indane.

L'invention concerne également un procédé de séparation de paraxylène de haute pureté.

L'invention concerne également un procédé de séparation de métaxylène de haute pureté.

### Liste des figures

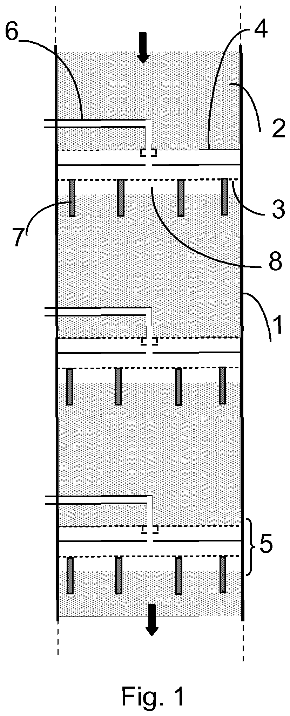

Les figures 1 à 3 illustrent l'invention et sont présentées à titre non limitatif.
Figure 1 : Schéma d'une colonne multi-étagée fonctionnant en lit mobile simulé

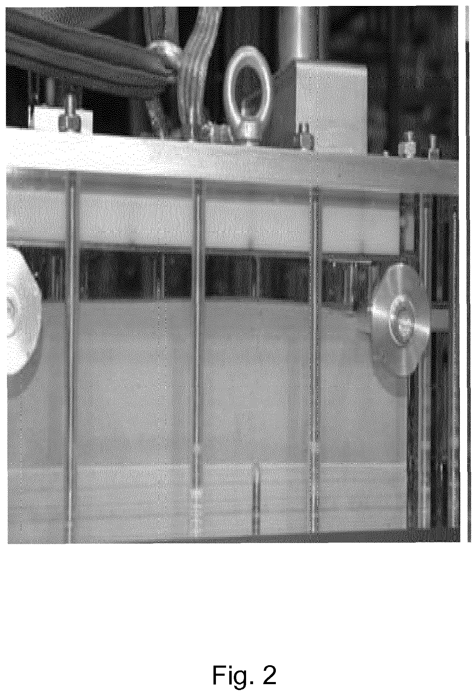

Figure 2 : Photographie des résultats obtenus en maquette froide pour le procédé de séparation des xylènes utilisant les formulations d'adsorbant selon l'invention A à D : absence de formation de sillons ou talus à la surface du lit granulaire à la vitesse superficielle linéaire maximale de 1,65cm/s (Exemple 2), après 4h sous écoulement, c'est-à-dire absence de déformation de la surface du lit granulaire par rapport à la ligne horizontale passant par la surface du lit au début de l'essai.

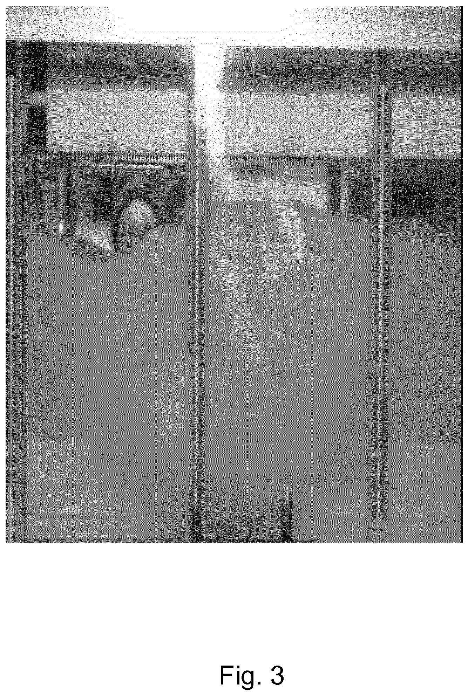

Figure 3 : Photographie des résultats obtenus en maquette froide pour le procédé de séparation des xylènes utilisant les formulations d'adsorbant E et F (essais comparatifs) : formation de sillons ou talus à la surface du lit granulaire pour des vitesses superficielles linéaires inférieures à 1,65 cm/s (Exemple 3) apparaissant dès les 20 premières minutes de l'essai. Sur la photographie prise après 4h sous écoulement, on observe une déformation de la surface du lit telle que les hauteurs du lit au point le plus bas et au point le plus haut du profil de hauteur de lit à la fin de l'essai diffère de respectivement 70% et 40% de la hauteur initiale du lit granulaire.

### Description sommaire de la figure 1

La figure 1 jointe représente une colonne multi-étagée avec plateaux distributeurs fonctionnant en lit mobile simulé. Cette figure est fournie à titre purement illustratif.

La colonne dans l'enceinte (1) est divisée en un certain nombre de lits granulaires (2). Entre deux lits granulaires successifs, notés lit amont et lit aval, s'intercale un plateau distributeur (5) posé sur des poutres (7). Une grille supérieure (4) supporte le milieu granulaire (2) tout en permettant au fluide de rentrer à l'intérieur du plateau. Les dispositifs de distribution comportent également un réseau de distribution (6) noyé dans le milieu granulaire (2), qui permet d'injecter ou de soutirer un fluide auxiliaire dans le plateau. En cas d'injection, le fluide auxiliaire injecté est ainsi mélangé avec le fluide principal venant du lit amont.

Le plateau distributeur (5) comporte également une grille inférieure (3) ou plaque perforée, ou tout autre moyen permettant de distribuer l'écoulement sur le lit granulaire aval. Il existe un espace vide (8) entre la grille inférieure (3) et la surface supérieure du lit granulaire aval.

### DESCRIPTION DETAILLEE DE L'INVENTION

L'invention concerne un procédé de séparation des xylènes à partir de coupes d'isomères C8 aromatiques en lit mobile simulé (LMS) consistant à utiliser comme solide adsorbant retenant sélectivement un isomère du xylène un adsorbant zéolithique aggloméré ayant en outre des caractéristiques particulières de granulométrie au niveau de la taille des adsorbants zéolithiques. Les particules de solide adsorbant zéolithique aggloméré utilisées dans le procédé selon l'invention ont un diamètre moyen (en nombre) des particules compris entre 150 µm et 500 µm, préférentiellement compris entre 200 µm et 400 µm. De préférence, les particules ont une répartition granulométrique telle qu'il n'existe aucune particule de taille inférieure à 100µm.

Plus particulièrement, on choisira, pour adsorber sélectivement :
(1) le paraxylène, des adsorbants zéolithiques à base de zéolithe X ou LSX, échangée au baryum (à au moins 90% exprimé en taux d'échange sur l'aggloméré final, estimé en évaluant le rapport entre le nombre de moles d'oxyde de baryum, BaO, et le nombre de moles de l'ensemble (BaO+Na₂O) de l'aggloméré final), ou échangée très majoritairement au baryum, et de manière minoritaire au potassium (le taux d'échange par les ions baryum et potassium étant au moins 90%, estimé en évaluant le rapport entre le nombre de moles de l'ensemble oxyde de baryum + oxyde de potassium (BaO+K₂O) et le nombre de moles de l'ensemble (BaO+K₂O+Na₂O)) de l'aggloméré final, ou (2) le métaxylène, des adsorbants zéolithiques à base de zéolithe Y, au sodium ou échangée au sodium et lithium tel que le rapport entre le nombre de moles d'oxyde de sodium Na₂O et le nombre de moles de l'ensemble oxyde de sodium + oxyde de lithium (Na₂O+Li₂O) de l'aggloméré final est supérieur à 65%, ce solide adsorbant ayant en outre les caractéristiques particulières de granulométrie au niveau de la taille des adsorbants zéolithiques.

Le procédé selon la présente invention peut être mis en œuvre aussi bien en phase liquide qu'en phase gazeuse.

L'invention concerne plus particulièrement un procédé de séparation de paraxylène ou de métaxylène à haute pureté (c'est à dire une pureté supérieure ou égale à 90%) en lit mobile simulé à partir d'une charge d'hydrocarbures aromatiques contenant des isomères à 8 atomes de carbones comprenant les étapes suivantes :
a) Une étape de mise en contact, dans des conditions d'adsorption adéquates, de la charge avec un lit contenant l'adsorbant sélectionné, de manière à adsorber préférentiellement le paraxylène ou le métaxylène,
b) une étape de mise en contact, dans des conditions de désorption, du lit d'adsorbant avec un désorbant, le désorbant étant préférentiellement soit du toluène, soit du paradiéthylbenzène,
c) une étape de soutirage du lit d'adsorbant d'un flux contenant le désorbant et les produits de la charge les moins sélectivement adsorbés,
d) une étape de soutirage du lit d'adsorbant d'un flux contenant le désorbant et le produit recherché, à savoir le paraxylène ou le métaxylène,
e) une séparation du flux issu de l'étape c) en un premier flux contenant le désorbant et un second flux contenant les produits de la charge les moins sélectivement adsorbés,
f) une séparation du flux issu de l'étape d) en un premier flux contenant le désorbant et un second flux contenant du paraxylène ou du métaxylène à un niveau de pureté supérieure ou égale à 90%, de manière préférée supérieure ou égale à 99%, et de manière très préférée supérieure ou égale à 99,7%.

Le procédé de séparation de paraxylène ou de métaxylène peut également optionnellement inclure les étapes suivantes :
g) une étape de cristallisation dans un cristalliseur consistant en la cristallisation du paraxylène issu de l'étape f), permettant d'obtenir d'une part des cristaux de paraxylène imbibés de leur liqueur mère, et d'autre part une liqueur mère qui peut être en partie, voire en totalité, recyclée en mélange avec la charge fraîche à l'entrée de l'unité d'adsorption en lit mobile simulé,
h) une étape de lavage des cristaux issus de l'étape g) à l'issue de laquelle on récupère du paraxylène ou du métaxylène à une pureté d'au moins 99,7%, et de manière préférée d'au moins 99,8%.

De manière plus précise l'objet de l'invention est d'optimiser le solide adsorbant mis en œuvre dans le procédé de séparation du paraxylène ou du métaxylène par adsorption en lit mobile simulé, pour maximiser la performance de ce procédé. En général, les performances recherchées pour la séparation d'une charge contenant des xylènes, sont une productivité maximale pour une pureté du produit recherché dans le flux d'extrait au moins égale à 99,5% et même 99,9%, et un rendement global du produit recherché au moins égal à 90%, voire supérieur à 95% et de façon préférée supérieur à 97%.

Dans la présente invention, le solide adsorbant utilisé est un adsorbant zéolithique à base de cristaux de zéolite, préférentiellement de zéolithe X, LSX ou Y, et éventuellement de phase non zéolithique (i.e. liant résiduel, phase amorphe, phases cristallines telle que quartz etc. après zéolithisation...), adsorbant dans lequel les cristaux ont un diamètre moyen en nombre inférieur ou égal à 1,2 µm, de préférence compris entre 0,1 µm et 1,2µm, et de manière préférée compris entre 0,5µm et 0,8µm.

Lorsque l'aggloméré selon la présente invention est préparé à partir de la zéolithe X ou LSX, le rapport atomique Si/Al est compris entre 1 et 1,5, de manière préférée compris entre 1,2 et 1,3, et l'aggloméré comprend :
i. Une teneur en oxyde de baryum BaO et une teneur en oxyde de potassium K₂O telle que le rapport entre le nombre de moles de l'ensemble oxyde de baryum + oxyde de potassium (BaO+K₂O) et le nombre de moles de l'ensemble (BaO+K₂O+Na₂O) est supérieur à 90%.
ii. Une teneur en oxyde de potassium K₂O telle que le rapport entre le nombre de mole d'oxyde de potassium K₂O et le nombre de mole d'oxyde de baryum BaO est inférieur à 50%.
iii.Et une teneur totale en oxydes d'ions alcalins ou alcalino-terreux autres que le baryum et le potassium de préférence inférieure à 5% et de préférence allant de 0 à 2% et avantageusement allant de 0 à 1% en poids par rapport au poids total de l'adsorbant zéolithique anhydre.

Lorsque l'adsorbant zéolithique selon la présente invention est préparé à partir de zéolithe Y, le rapport atomique Si/Al est compris entre 1,5 et 6, de manière préférée compris entre 2,5 et 3, et l'adsorbant zéolithique comprend
i. Une teneur en oxyde de sodium Na₂O et une teneur en oxyde de lithium Li₂O telle que le rapport entre le nombre de moles d'oxyde de sodium Na₂O et le nombre de moles de l'ensemble oxyde de sodium + oxyde de lithium (Na₂O+Li₂O) est supérieur à 65%.
ii. Et une teneur totale en oxydes d'ions alcalins ou alcalino-terreux autres que le sodium et le lithium de préférence inférieure à 5% et de préférence allant de 0 à 2% et avantageusement allant de 0 à 1% en poids par rapport au poids total de l'adsorbant zéolithique anhydre.

Les adsorbants zéolithiques selon l'invention ont un diamètre moyen (en nombre) des particules compris entre 150 µm et 500 µm, préférentiellement compris entre 200 µm et 400 µm et de préférence avec une répartition granulométrique telle qu'il n'existe aucune particule de taille inférieure à 100µm.

Lorsque les adsorbants zéolithiques selon la présente invention sont préparés à partir de la zéolithe X ou LSX, ils ont de préférence une densité de grain comprise entre 1,1 et 1,4 g/mL, et de manière préférée entre 1,1 et 1,3g/mL telle que mesurée par intrusion de mercure (exprimée par rapport à la masse sèche de l'adsorbant zéolithique) et un volume poreux mesuré par intrusion de mercure (volume poreux contenu dans les macropores et les mésopores de diamètre apparent supérieur à 4 nm) compris entre 0,20 et 0,35 mL/g (exprimé par rapport à la masse sèche de l'adsorbant zéolithique).

Les adsorbants zéolithiques utilisés dans la présente invention sont obtenus classiquement selon un procédé comprenant les étapes suivantes:
1/ mélange des cristaux de zéolithe X, LSX ou Y en poudre de granulométrie souhaitée, en présence d'eau avec au moins un liant à base d'une argile ou d'un mélange d'argiles,
2/ mise en forme du mélange obtenu en 1/ pour produire des agglomérés, éventuellement suivie d'une étape de tamisage et/ou de cyclonage,
3/ calcination des agglomérés obtenus en 2/ à une température allant de 500°C à 600°C,
4/ (éventuellement) zéolithisation du liant par mise en contact du produit résultant de 3/ avec une solution aqueuse basique alcaline suivie d'un lavage ;
5/ échange ionique des agglomérés zéolithiques à base de zéolithe X ou LSX obtenus en 3/ ou en 4/ par des ions baryum seuls soit par des ions baryum et des ions potassium et éventuellement échange ionique partiel des agglomérés zéolithiques à base de zéolithe Y au sodium obtenus en 3/ ou en 4/ par des ions lithium, suivi d'un lavage et d'un séchage du produit ainsi traité ;
6/ activation du produit issu de l'étape 5 à une température allant de 200 à 300°C.

L'étape 2/ de mise en forme permet d'obtenir des agglomérés zéolithiques présentant une résistance mécanique suffisante pour leur utilisation dans un procédé de séparation des xylènes en lit mobile simulé. Cependant la présence de liant réduit la proportion de matière active au sens de l'adsorption (zéolithe X, LSX ou Y).

L'étape optionnelle 4/ de zéolithisation du liant permet ainsi de transformer tout ou partie du liant en matière active au sens de l'adsorption (zéolithe X, LSX ou Y) afin d'obtenir des agglomérés sans liant ("binderless"), i.e. ne comportant plus de phase non zéolithique ou en quantité typiquement inférieure à environ 1% ou des agglomérés "binderlow", i.e. comportant peu de phase non zéolithique, c'est-à-dire généralement du liant résiduel non zéolithisé ou toute autre phase amorphe après zéolithisation, en quantité typiquement comprise entre environ 2% et 5% dans l'aggloméré final, et ce, tout en maintenant la résistance mécanique. Le taux de phase non zéolithique (i.e. liant résiduel non zéolithisé, phase amorphe, après zéolithisation) dans l'aggloméré final peut être quantifié par référence à un adsorbant composé uniquement de zéolithe, sous forme de poudre, à partir de mesures d'adsorption ou à partir d'intensités de pics de DRX. La zéolithe de référence est la zéolithe utilisée à l'étape 1/ du procédé d'obtention de l'adsorbant, et ayant subi le même échange ionique.

Les cristaux de zéolithes X, LSX ou Y issus de la zéolithisation du liant (transformation du liant en zéolithe) sont généralement de plus petits diamètres que les cristaux initiaux. Par conséquent, dans l'aggloméré final, les cristaux dont le diamètre moyen est inférieur ou égal à 1,2 µm, de préférence compris entre 0,1 µm et 1,2 µm, et de manière préférée entre 0,5 µm et 0,8 µm, présentent de manière classique une distribution granulométrique monomodale, mais on ne sort pas du cadre de l'invention si la distribution des diamètres des cristaux est multi-modale, et en particulier bi-modale, du fait de la présence de la population des cristaux issus de la zéolithisation du liant.

La performance des adsorbants zéolithiques, tels que décrits précédemment, pour le procédé de séparation du paraxylène ou du métaxylène, en terme de pureté de paraxylène ou de métaxylène dans l'extrait et de rendement du procédé, est influencée par différents paramètres du procédé, à savoir les conditions opératoires, la composition de la charge d'alimentation, la teneur en eau et le type de désorbant. Les conditions opératoires de l'unité industrielle d'adsorption à contre-courant simulé mettant en œuvre les adsorbants zéolithiques tels que décrits précédemment, sont typiquement les suivantes :
- nombre de lits compris entre 4 et 24
- nombre de zones : au moins 4
- température 100°C à 250°C, de préférence 150°C à 190°C,
- pression comprise entre la pression de bulle des xylènes à la température du procédé et 3 MPa
- temps de cycle, correspondant au temps entre deux injections de désorbant sur un lit donné, compris entre 4 et 18 min
- rapport des débits désorbant sur charge 0,7 à 2,5
- taux de recyclage (rapport du débit moyen des zones pondéré du nombre de lits présents dans chaque zone sur le débit de charge) de 2 à 12, de préférence 2,5 à 4.

Le désorbant est préférentiellement soit du toluène, soit du paradiéthylbenzène lorsque le composé à séparer est le paraxylène et préférentiellement soit du toluène, soit de l'indane lorsque le composé à séparer est le métaxylène.

La teneur en eau dans les effluents hydrocarbonés est préférentiellement ajustée entre 20 ppm et 150 ppm pour les adsorbants à base de zéolithe X et LSX pour une température du procédé de 165°C à 185°C, afin d'obtenir des résultats optimum en productivité.

La teneur en eau dans les effluents hydrocarbonés est préférentiellement ajustée entre 0 et 80 ppm pour les adsorbants à base de zéolithe Y pour une température du procédé de 120°C à 180°C, afin d'obtenir des résultats optimum en productivité.

### Techniques de caractérisation des adsorbants zéolithiques

L'estimation du diamètre moyen en nombre des solides adsorbants obtenus à l'issus de l'étape 2/ de mise en forme nécessite d'effectuer une analyse de la distribution granulométrique d'un échantillon d'adsorbant par imagerie selon la norme ISO 13322 - 2 : 2006 en utilisant un tapis roulant permettant à l'échantillon de passer devant l'objectif de la caméra.

Le diamètre moyen en nombre est ensuite calculé à partir de la distribution granulométrique en appliquant la norme ISO 9276-2:2001. Dans le présent document, on emploie l'appellation « diamètre moyen en nombre » ou bien « taille » pour les particules d'adsorbants zéolithiques. La précision est de l'ordre de 10 µm pour la gamme de taille d'adsorbants zéolithiques de l'invention.

L'estimation du diamètre moyen en nombre des cristaux de zéolite X, LSX ou Y contenue dans les adsorbants zéolithiques est réalisée par observation au microscope électronique à balayage (MEB) . Afin d'estimer la taille des cristaux de zéolithe sur les échantillons, on effectue un ensemble de clichés à un grossissement d'au moins 5000. On mesure ensuite le diamètre d'au moins 200 cristaux à l'aide de logiciels dédiés, par exemple le logiciel Smile View de l'éditeur LoGraMi). Le diamètre moyen en nombre est ensuite calculé à partir de la distribution granulométrique en appliquant la norme ISO 9276-2:2001.

On emploie l'appellation « diamètre moyen en nombre » ou bien « taille » pour les cristaux de zéolite. La précision est de l'ordre de 3 %.

L'observation des adsorbants zéolithiques au MEB permet également de confirmer la présence de phase non zéolithique comprenant par exemple du liant résiduel (non converti lors de l'étape de zéolithisation) ou toute autre phase amorphe dans les agglomérés.

Une analyse chimique élémentaire de l'adsorbant peut être réalisée par différentes techniques analytiques connues par l'homme du métier. Parmi ces techniques on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société BRUKER.

La fluorescence X est une technique spectrale qui offre une détermination précise, à la fois quantitative et qualitative, sauf pour les éléments les plus légers tels que le lithium, le sodium ou le potassium présents en très faibles teneurs. On préférera dans ce cas la spectrométrie d'émission atomique avec plasma induit par haute fréquence (ICP-AES) décrites dans la norme NF EN ISO 21079-3 sur un appareil de type par exemple Perkin Elmer 4300DV.

Ces analyses chimiques élémentaires permettent à la fois de vérifier le rapport atomique Si/Al de l'adsorbant zéolithique, et de mesurer les teneurs en oxydes d'ions alcalins ou alcalino-terreux et notamment BaO, K₂O, Na₂O, Li₂O.

A noter que les teneurs en différents oxydes sont données quelle que soit la technique en pourcent poids par rapport au poids total de l'adsorbant anhydre.

La technique d'intrusion de mercure est utilisée pour caractériser le volume poreux intra-granulaire contenu dans les pores de diamètres supérieurs à 3,6 nm de l'adsorbant, et pour mesurer sa densité de grain. Un porosimètre à mercure type Autopore 9500 Microméritics est utilisé pour analyser la répartition du volume poreux contenu dans les macropores de diamètre de pore > 50nm et dans les mésopores compris entre 3,6 et 50 nm. Le volume microporeux au sein des cristaux de zéolithe ainsi que le volume poreux contenu dans les petits mésopores compris entre 2 et 3,6nm ne sont pas accessibles avec les porosimètres actuels. La méthode expérimentale, décrite dans le manuel opératoire de l'appareil, consiste à placer un échantillon d'adsorbant (de perte au feu connue) préalablement pesé, dans une cellule du porosimètre, puis, après un dégazage préalable (pression d'évacuation de 30µm Hg pendant au moins 10 min), à remplir la cellule avec du mercure à une pression donnée (0,0036MPa), et ensuite à appliquer une pression croissante par palier jusqu'à 400 MPa afin de faire pénétrer progressivement le mercure dans le réseau poreux de l'échantillon. La relation entre la pression appliquée et le diamètre des pores est établie en supposant des pores cylindriques, un angle de contact entre le mercure et la paroi des pores de 140° et une tension de surface du mercure de 485 dynes /cm.

La quantité cumulée de mercure introduite en fonction de la pression appliquée est enregistrée. L'appareil ne permet pas de différencier le volume inter-granulaire et le volume intra-granulaire : on suppose qu'à environ 0,2MPa (correspondant à des diamètres apparents de pores de 7µm), le mercure remplit tous les vides intergranulaires, et qu'au delà le mercure pénètre dans les pores de l'adsorbant. La densité de grain de l'adsorbant est ainsi calculée en divisant la masse de l'échantillon par le volume de l'échantillon évalué d'après le volume de mercure introduit à une pression de 0,2 MPa.

Le volume poreux total de l'adsorbant est ensuite évalué à partir du volume total de mercure introduit corrigé du volume de mercure introduit à une pression de 0.2MPa. Dans le présent document, la densité de grain et le volume poreux des adsorbants zéolithiques mesurés par intrusion de mercure sont rapportés à la masse de l'échantillon anhydre (par correction de la perte au feu de l'échantillon analysé).

La technique de caractérisation de la résistance mécanique représentative de l'écrasement de l'adsorbant au sein d'un lit ou d'un réacteur est la technique de caractérisation de la résistance mécanique en lit, telle que décrite dans la méthode Shell série SMS1471-74 (Shell Method Series SMS1471-74 Détermination of Bulk Crushing Strength of Catalysts. Compression-Sieve Method"), associée à l'appareil "BCS Tester" commercialisé par la société Vinci Technologies. Cette méthode, initialement destinée à la caractérisation de catalyseurs de 3 à 6 mm est basée sur l'utilisation d'un tamis de 425 µm qui va permettre notamment de séparer les fines créées lors de l'écrasement. L'utilisation d'un tamis de 425 µm reste adaptée pour des particules de diamètre supérieur à 1,6mm, mais doit être adaptée selon la granulométrie des adsorbants zéolithiques que l'on cherche à caractériser. La norme ASTM D7084-04 qui décrit également une méthode de mesure de la résistance à l'écrasement en lit de catalyseurs ("Détermination of Bulk Crush Strength of Catalysts and Catalyst Carriers") définit le passage du tamis à utiliser comme étant égal à la moitié du diamètre des particules de catalyseurs à caractériser. La méthode prévoit une étape préliminaire de tamisage de l'échantillon de catalyseurs ou adsorbants à caractériser. Si une quantité égale à 10% pds de l'échantillon passe à travers la grille, un tamis de passage plus petit sera utilisé.

Les adsorbants zéolithiques de la présente invention sont sous forme de billes et ont un diamètre moyen en nombre allant de 150 µm à 500 µm, et de préférence allant de 200 µm à 400 µm. De manière préférée, aucune particule n'a une taille inférieure à 100 µm. Par conséquent, un tamis de 100 µm sera utilisé à la place du tamis de 425µm mentionnée dans la méthode Shell standard SMS1471-74.

La méthode se déroule de la manière suivante : un échantillon de 20 cm³ d'adsorbants agglomérés, préalablement tamisé avec le tamis adapté (100 µm) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm3 de billes d'acier afin de mieux répartir la force exercée par le piston sur les agglomérés d'adsorbants (utilisation de billes de 4 mm de diamètre pour l'analyse de tous les types d'extrudés et les particules de forme sphérique ayant un diamètre > 1.6 mm, et de billes de 2 mm de diamètre pour des particules de forme sphérique de diamètre < 1.6 mm). Les fines obtenues aux différents paliers de pression sont séparées par tamisage (tamis adapté de 100 µm) et pesées. La résistance à l'écrasement en lit est déterminée par la pression en méga Pascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 et 3,2 MPa.

La précision est de manière classique inférieure à 0,1 MPa.

### EXEMPLES

Exemple 1 : Formulation de référence Ref. (comparatif) : Dans cet exemple, un adsorbant zéolithique à base de BaX formant des cristaux de 1,5 µm mis en forme en billes de 0,6 mm de diamètre est utilisé pour la séparation de paraxylène en lit mobile simulé L'adsorbant est préparé selon le procédé de fabrication décrit dans la demande de brevet WO08/009845).

On utilise une unité fonctionnant en lit mobile simulé constituée de 24 lits, de longueur 1,1m, avec une injection de charge, une injection de désorbant, un soutirage d'extrait et un soutirage de raffinat. Les lits sont répartis en 4 zones chromatographiques selon la configuration : 5 / 9 / 7 / 3.

La charge est composée de 21,6 % de paraxylène, de 20,8 % d'orthoxylène, de 47,9 % de métaxylène et de 9,7 % d'éthylbenzène. Le désorbant est du paradiéthylbenzène. La température est de 175°C, et la pression de 15 bars. La teneur en eau est de 95 ppm (poids).

La productivité de référence est de 70 kg de paraxylène / m³/h .

La vitesse superficielle linéaire en zone 3 est de 1,63 cm/s.

### Exemple 2 : Formulations A à D (selon l'invention)

Les performances des différentes formulations selon l'invention en lit mobile simulé sont évaluées et exprimées en gain en productivité par rapport à l'exemple de référence précédent. L'objectif de vitesse superficielle linéaire maximale en zone 3 est maintenu à 1,65 cm /s.
**Formulation A :** billes de 300µm constituées de cristaux de 1,2µm
**Formulation B** : billes de 300µm constituées de cristaux de 0,3µm
**Formulation C** : billes de 200µm constituées de cristaux de 0,8µm
**Formulation D** : billes de 200µm constituées de cristaux de 0,5µm

Par ailleurs des essais en maquette reproduisant une section d'un adsorbeur (élément de distribution supérieur, lit d'adsorbant et élément de distribution inférieur) ont été réalisés avec les formulations A à D afin d'observer l'éventuelle formation de sillons ou talus à la surface du lit granulaire, qui est préjudiciable pour la productivité à long terme.

Pour l'ensemble des formulations A à D, on n'observe pas de formation de sillons ou talus à la surface du lit granulaire à la vitesse superficielle linéaire maximale de 1,65cm/s (Figure 2), c'est-à-dire que sur les clichés on n'observe pas de déformation de la surface du lit granulaire par rapport à la ligne horizontale passant par la surface du lit au début de l'essai.

### Exemple 3 : Formulations E et F (comparatif)

Des formulations de granulométrie plus petite que celle des exemples A à D sont testées et le gain en productivité par rapport à l'exemple comparatif est évalué. L'objectif de vitesse superficielle linéaire maximale en zone 3 est maintenu à 1,65 cm /s.
**Formulation E** : billes de 100µm constituées de cristaux de 1,2µm
**Formulation F** : billes de 100µm constituées de cristaux de 0,5µm

Des essais en maquette ont également été réalisés avec les formulations E et F, et montrent la formation de sillons ou talus à la surface du lit granulaire pour des vitesses superficielles linéaires inférieures à 1,65 cm/s (Figure 3). Après 4h sous écoulement, on observe une déformation de la surface du lit telle que la hauteur du lit au point le plus bas et/ou au point le plus haut du profil de hauteur de lit à la fin de l'essai diffère d'au moins 10% de la hauteur initiale du lit granulaire.

### Exemple 4 : Résultats

Le tableau 1 présente les résultats du test en maquette sur les différentes formulations A, B, B, C, D, E, F par rapport à la référence Ref.

Il apparaît que le choix d'une granulométrie selon l'invention pour les particules de solide adsorbant, en association avec une faible taille de cristaux de zéolithes, permet d'obtenir une bonne productivité initiale, tout en évitant la formation de talus, ce qui permet de maintenir cette productivité à long terme.

| | diamètre des billes (µm) | diamètre des cristaux (µm) | Résistance mécanique à l'écrasement en lit (MPa) | vitesse en zone 3 (cm/s) | gain de producti vité initiale / Ref. | Formation de talus et perte de productivit é à long terme |
|---|---|---|---|---|---|---|
| Ref. (comparatif) | 600 | 1.5 | 2.4 | 1.63 | | Non |
| A | 300 | 1.2 | 2.5 | 1.28 | 2.4 | Non |
| B | 300 | 0.3 | 2.0 | 1.60 | 6.7 | Non |
| C | 200 | 0.8 | 2.3 | 1.63 | 5.9 | Non |
| D | 200 | 0.5 | 2.1 | 1.66 | 9.0 | Non |
| E (comparatif) | 100 | 1.2 | - | 1.50 | 3.7 | Oui |
| F (comparatif) | 100 | 0.5 | - | 1.65 | 9.2 | Oui |

## Revendications

1. Procédé de séparation des xylènes à partir d'une charge comprenant des coupes d'isomères d'hydrocarbures aromatiques contenant 8 atomes de carbone, en lit mobile simulé, par adsorption sélective d'un isomère du xylène en présence d'un désorbant, au moyen de particules d'adsorbant zéolithique aggloméré, sous forme de billes, à base de cristaux de zéolithe de diamètre moyen en nombre inférieur ou égal à 1,2 µm, dans lequel le diamètre moyen en nombre desdites particules d'adsorbant est compris entre 150 µm et 500 µm et la résistance mécanique mesurée par la méthode Shell série SMS1471-74 adaptée pour les agglomérés de taille inférieure à 500 µm, c'est-à-dire utilisant un échantillon tamisé avec un tamis de 100µm et séché à une température de 250°C, est supérieure ou égale à 2 MPa,
le temps de cycle, correspondant au temps entre deux injections de désorbant sur un lit donné, étant compris entre 4 et 18 min et
le rapport des débits de désorbant sur charge étant compris entre 0,7 et 2,5 et le taux de recyclage est compris entre 2,0 et 12.

2. Procédé de séparation des xylènes selon la revendication 1 dans lequel le diamètre moyen en nombre desdites particules d'adsorbant est compris entre 200 µm et 400 µm.

3. Procédé de séparation des xylènes selon l'une des revendications 1 ou 2 dans lequel la répartition granulométrique desdites particules d'adsorbant est telle qu'il n'existe aucune particule de taille inférieure à 100µm.

4. Procédé de séparation des xylènes selon l'une des revendications 1 à 3 dans lequel le diamètre moyen en nombre des cristaux de zéolithe est compris entre 0,1 µm et 1,2 µm.

5. Procédé de séparation des xylènes selon la revendication 4 dans lequel le diamètre moyen en nombre des cristaux de zéolithe est compris entre 0,5 µm et 0,8 µm.

6. Procédé de séparation des xylènes selon l'une des revendications précédentes mis en œuvre dans une unité en lit mobile simulé ayant les caractéristiques suivantes:
- nombre de lits compris entre 4 et 24
- nombre de zones : au moins 4.

7. Procédé de séparation des xylènes selon l'une des revendications précédentes opérant à une température de 100°C à 250°C, et à une pression comprise entre la pression de bulle des xylènes à la température du procédé et 3 MPa.

8. Procédé de séparation du paraxylène selon l'une des revendications 1 à 7 par adsorption sélective du paraxylène dans lequel l'adsorbant zéolithique aggloméré est à base de zéolithe X ou LSX ayant un rapport atomique Si/Al tel que 1,0 ≤ Si/Al < 1,5.

9. Procédé de séparation du paraxylène selon la revendication 8 dans lequel l'adsorbant zéolithique aggloméré comprend en outre :
i. Une teneur en oxyde de baryum BaO et une teneur en oxyde de potassium K₂O telle que le rapport entre le nombre de moles de l'ensemble oxyde de baryum + oxyde de potassium (BaO+K₂O) et le nombre de moles de l'ensemble (BaO+K₂O+Na₂O) est supérieur à 90% ;
ii. Une teneur en oxyde de potassium K₂O telle que le rapport entre le nombre de mole d'oxyde de potassium K₂O et le nombre de mole d'oxyde de baryum BaO est inférieur à 0,5 ;
iii. et une teneur totale en oxydes d'ions alcalins ou alcalino-terreux autres que le baryum et le potassium de préférence inférieure à 5% par rapport au poids total de l'adsorbant zéolithique.

10. Procédé de séparation du paraxylène selon la revendication 8 ou 9 dans lequel l'adsorbant zéolithique aggloméré a une densité de grain comprise entre 1,1 et 1,4 g/mL, telle que mesurée par intrusion de mercure (exprimé par rapport à la masse sèche de l'adsorbant zéolithique) et un volume poreux total mesuré par intrusion de mercure (volume poreux contenu dans les macropores et les mésopores de diamètre apparent supérieur à 4 nm) compris entre 0,20 et 0,35 mL/g (exprimé par rapport à la masse sèche de l'adsorbant zéolithique).

11. Procédé de séparation du paraxylène selon l'une des revendications 8 à 10 mis en œuvre à une température de 165°C à 185°C et dans lequel la teneur en eau dans les effluents hydrocarbonés est ajustée entre 20 ppm et 150 ppm, en ajoutant de l'eau dans la charge comprenant les coupes d'isomères d'hydrocarbures aromatiques contenant 8 atomes de carbone et/ou dans le désorbant.

12. Procédé de séparation du paraxylène selon l'une des revendications 8 à 11, dans lequel le désorbant est choisi parmi le toluène et le paradiéthylbenzène.

13. Procédé de séparation du métaxylène selon l'une des revendications 1 à 7 par adsorption sélective du métaxylène, dans lequel l'adsorbant zéolithique aggloméré est à base de zéolithe Y ayant un rapport atomique Si/Al tel que 1,5 < Si/Al < 6.

14. Procédé de séparation du métaxylène selon la revendication 13 dans lequel l'adsorbant zéolithique aggloméré comprend en outre :
i. Une teneur en oxyde de sodium Na₂O et une teneur en oxyde de lithium Li₂O telle que le rapport entre le nombre de moles d'oxyde de sodium et le nombre de moles de l'ensemble oxyde de sodium + oxyde de lithium (Na₂O+Li₂O) est supérieur à 65%.
ii. Et une teneur totale en oxydes d'ions alcalins ou alcalino-terreux autres que le sodium et le lithium de préférence inférieure à 5% par rapport au poids total de l'adsorbant zéolithique.

15. Procédé de séparation du métaxylène selon l'une des revendications 13 ou 14 mis en œuvre à une température de 120°C à 180°C et dans lequel la teneur en eau dans les effluents hydrocarbonés est ajustée entre 0 ppm et 80 ppm, en ajoutant de l'eau dans la charge comprenant les coupes d'isomères d'hydrocarbures aromatiques contenant 8 atomes de carbone et/ou dans le désorbant.

16. Procédé de séparation du métaxylène selon l'une des revendications 13 à 15, dans lequel le désorbant est choisi parmi le toluène et l'indane.

## Patentansprüche

1. Verfahren zur Separation von Xylolen aus einer aromatische Kohlenwasserstoffisomerfraktionen umfassenden Ladung, die 8 Kohlenstoffatome enthält, auf einem simulierten Bewegbett durch selektives Adsorbieren eines Xylolisomers in Gegenwart eines Desorptionsmittels mittels Teilchen eines agglomerierten zeolithischen Adsoptionsmittels in Kugelform, das auf Zeolithkristallen mit einem zahlengemittelten Durchmesser kleiner oder gleich 1,2 µm basiert, wobei der zahlengemittelte Durchmesser der Adsorptionsmittelteilchen zwischen 150 und 500 µm liegt und die im auf Agglomerate kleiner 500 µm angepassten Shell-Verfahren (Ifd. Nr. SMS1471-74), d.h. unter Verwendung einer mit einem 100-µm-Sieb gesiebten, bei einer Temperatur von 250 °C getrockneten Probe, gemessene mechanische Festigkeit mindestens 2 MPa beträgt,
wobei die Zykluszeit, die der Zeit zwischen zwei Injektionen von Desorptionsmittel in ein jeweiliges Bett entspricht, zwischen 4 und 18 min liegt und
das Durchflussverhältnis des Desorptionsmittels bei einer jeweiligen Ladung zwischen 0,7 und 2,5 liegt und die Rezyklisierungsrate zwischen 2,0 und 12 liegt.

2. Verfahren zur Separation von Xylolen nach Anspruch 1, wobei der zahlengemittelte Durchmesser der Adsorptionsmittelteilchen zwischen 200 und 400 µm liegt.

3. Verfahren zur Separation von Xylolen nach einem der Ansprüche 1 oder 2, wobei die Korngrößenverteilung der Adsorptionsmittelteilchen derart ist, dass kein Teilchen kleiner 100 µm vorhanden ist.

4. Verfahren zur Separation von Xylolen nach einem der Ansprüche 1 - 3, wobei der zahlengemittelte Durchmesser der Zeolithkristalle zwischen 0,1 und 1,2 µm liegt.

5. Verfahren zur Separation von Xylolen nach Anspruch 4, wobei der zahlengemittelte Durchmesser der Zeolithkristalle zwischen 0,5 und 0,8 µm liegt.

6. Verfahren zur Separation von Xylolen nach einem der vorstehenden Ansprüche, das in einer Anlage mit simuliertem Bewegett mit folgenden Eigenschaften durchgeführt wird:
- Anzahl der Bette zwischen 4 und 24,
- Anzahl der Bereiche: mindestens 4.

7. Verfahren zur Separation von Xylolen nach einem der vorstehenden Ansprüche, das bei einer Temperatur von 100 - 250 °C und einem Druck zwischen dem Blasendruck der Xylole bei der Verfahrenstemperatur und 3 MPa durchgeführt wird..

8. Verfahren zur Separation von Paraxylol nach einem der Ansprüche 1 - 7 durch selektive Adsorption von Paraxylol, wobei das agglomerierte zeolitische Adsorptionsmittel auf Zeolith X oder LSX basiert und ein derartiges Atomverhältnis von Si zu Al aufweist, dass 1,0 ≤ Si/Al < 1,5.

9. Verfahren zur Separation von Paraxylol nach Anspruch 8, wobei das agglomerierte zeolithische Adsorptionsmittel ferner umfasst:
i. Einen Bariumoxidgehalt (BaO) und einen Kaliumoxidgehalt (K₂O), sodass das Verhältnis zwischen der Anzahl Mole von Bariumoxid und Kaliumoxid zusammen (BaO+K₂O) und der Anzahl Mole von BaO+K₂O+Na₂O) 90% übersteigt;
ii. Einen Kaliumoxidgehalt (K₂O), sodass das Verhältnis zwischen der Anzahl Mole von Kaliumoxid K₂O und der Anzahl Mole von Bariumoxid BaO kleiner 0,5 ist;
iii. und einen Gesamtgehalt an Oxiden von alkalischen oder erdalkalischen Ionen außer Barium und Kalium, der, bezogen auf das Gesamtgewicht des zeolithischen Adsorptionsmittels, vorzugsweise kleiner 5 % ist.

10. Verfahren zur Separation von Paraxylol nach Anspruch 8 oder 9, wobei das agglomerierte zeolitische Adsorptionsmittel eine mittels Quecksilberintrusion gemessene Korndichte zwischen 1,1 und 1,4 g/mL aufweist (bezogen auf die Trockenmasse des zeolithischen Adsorptionsmittels), und ein mittels Quecksilberintrusion gemessenes Porenvolumen (Porenvolumen der Makroporen und mittelgroßen Poren mit einem scheinbaren Durchmesser größer 4 nm) zwischen 0,20 und 0,35 mL/g insgesamt (bezogen auf die Trockenmasse des zeolithischen Adsorptionsmittels) aufweist.

11. Verfahren zur Separation von Paraxylol nach einem der Ansprüche 8 - 10, das bei einer Temperatur von 165 - 185°C durchgeführt wird, wobei der Wassergehalt der kohlenwasserstoffhaltigen Abwässer zwischen 20 und 150 ppm eingestellt wird, indem der Ladung, die die 8 Kohlenstoffatome enthaltenden aromatischen Kohlenwasserstoffisomerfraktionen umfasst, und/oder dem Desorptionsmittel Wasser beigegeben wird.

12. Verfahren zur Separation von Paraxylol nach einem der Ansprüche 8-11, wobei das Desorptionsmittel aus Toluol und Paradiethylbenzol gewählt ist.

13. Verfahren zur Separation von Metaxylol nach einem der Ansprüche 1 - 7 durch selektive Adsorption von Metaxylol, wobei das agglomerierte zeolitische Adsorptionsmittel auf Zeolith Y basiert und ein derartiges Atomverhältnis von Si zu Al aufweist, dass 1,5 ≤ Si/Al < 6.

14. Verfahren zur Separation von Metaxylol nach Anspruch 13, wobei das agglomerierte zeolithische Adsorptionsmittel ferner umfasst:
i. Einen Natriumoxidgehalt (Na₂O) und einen Lithiumoxidgehalt (Li₂O), sodass das Verhältnis zwischen der Anzahl Mole von Natriumoxid und der Anzahl Mole von Natriumoxid + Lithiumoxid (Na₂O+Li₂O) größer 65 % ist.
ii. und einen Gesamtgehalt an Oxiden von alkalischen oder erdalkalischen Ionen außer Natrium und Lithium, der, bezogen auf das Gesamtgewicht des zeolithischen Adsorptionsmittels, vorzugsweise kleiner 5 % ist.

15. Verfahren zur Separation von Metaxylol nach einem der Ansprüche 13 oder 14, das bei einer Temperatur von 120 - 180°C durchgeführt wird, wobei der Wassergehalt der kohlenwasserstoffhaltigen Abwässer zwischen 0 und 80 ppm eingestellt wird, indem der Ladung, die die 8 Kohlenstoffatome enthaltenden aromatischen Kohlenwasserstoffisomerfraktionen umfasst, und/oder dem Desorptionsmittel Wasser beigegeben wird.

16. Verfahren zur Separation von Metaxylol nach einem der Ansprüche 13-15, wobei das Desorptionsmittel aus Toluol und Indan gewählt ist.

## Claims

1. A process for separating xylenes starting from a feed comprising cuts of isomers of aromatic hydrocarbons containing 8 carbon atoms, the process comprising selectively adsorbing a xylene isomer from the feed in a simulated moving bed in the presence of a desorbent, using particles of agglomerated zeolitic adsorbent, in the form of beads, based on zeolite crystals with a number-average diameter less than or equal to 1.2 µm, wherein the number-average diameter of said particles of adsorbent is between 150 µm and 500 µm and the mechanical strength measured by the Shell method series SMS1471-74 adapted for agglomerates with a size below 500 µm, i.e. using a sieved sample with a 100 µm sieve and dried at a temperature of 250°C, is greater than or equal to 2 MPa,
the cycle time, corresponding to the time between two injections of desorbent on a given bed, being between 4 and 18 min,
the ratio of the flow rates of desorbent to feed being between 0.7 and 2.5 and the recycling rate being between 2.0 and 12.

2. The process for separating xylenes according to claim 1, wherein the number-average diameter of said particles of agglomerated zeolitic adsorbent is between 200 µm and 400 µm.

3. The process for separating xylenes according to claim 1 or 2, wherein the granulometric distribution of said particles of adsorbent is such that there is no particle with size less than 100 µm.

4. The process for separating xylenes according to one of claims 1 to 3, wherein the number-average diameter of the zeolite crystals is between 0.1 µm and 1.2 µm.

5. The process for separating xylenes according to claim 4, wherein the number-average diameter of the zeolite crystals is between 0.5 µm and 0.8 µm.

6. The process for separating xylenes according to one of preceding claims, wherein the process is carried out in a simulated moving-bed unit having the following characteristics:
- number of beds between 4 and 24
- number of zones: at least 4.

7. The process for separating xylenes according to one of preceding claims, wherein the adsorption is carried out at a temperature from 100° C. to 250° C., and at a pressure between the bubble pressure of the xylenes at the process temperature and 3 MPa.

8. The process for separating xylenes according to one of claims 1 to 7, wherein the xylene which is selectively adsorbed is para-xylene and wherein the agglomerated zeolitic adsorbent is based on zeolite X or LSX having an Si/AI atomic ratio such that 1.0≦Si/Al<1.5.

9. The process for separating xylenes according to claim 8, wherein the agglomerated zeolitic adsorbent further comprises:
i. a content of barium oxide BaO and a content of potassium oxide K₂O such that the ratio of the number of moles of the total barium oxide+potassium oxide (BaO+K₂O) to the number of moles of the total (BaO+K₂O+Na₂O) is greater than 90%;
ii. a content of potassium oxide K₂O such that the ratio of the number of moles of potassium oxide K₂O to the number of moles of barium oxide BaO is less than 0.5; and
iii. a total content of oxides of alkali-metal or alkaline-earth ions other than barium and potassium preferably below 5% relative to the total weight of the agglomerated zeolitic adsorbent.

10. The process for separating xylenes according to claim 8 or 9, wherein the agglomerated zeolitic adsorbent has a grain density between 1.1 and 1.4 g/mL, as measured by mercury intrusion (expressed relative to the dry mass of the zeolitic adsorbent) and a total pore volume measured by mercury intrusion (pore volume contained in the macropores and the mesopores with apparent diameter greater than 4 nm) between 0.20 and 0.35 mL/g (expressed relative to the dry mass of the zeolitic adsorbent).

11. The process for separating xylenes according to one of claims 8 to 10, wherein the process carried out at a temperature from 165° C. to 185° C. and wherein the water content of the hydrocarbon effluents is adjusted between 20 ppm and 150 ppm, by adding water to the feed comprising the cuts of isomers of aromatic hydrocarbons containing 8 carbon atoms and/or to the desorbent.

12. The process for separating xylenes according to one of claims 8 to 10, wherein the desorbent is selected from the group consisting of toluene and para-diethylbenzene.

13. The process for separating xylenes according to one to claims 1 to 7, wherein meta-xylene is selectively adsorbed and wherein the agglomerated zeolitic adsorbent is based on zeolite Y having an Si/AI atomic ratio such that 1.5<Si/AI<6.

14. The process for separating xylenes according to claim 13, wherein the agglomerated zeolitic adsorbent further comprises:
i. a content of sodium oxide Na₂O and a content of lithium oxide Li₂O such that the ratio of the number of moles of sodium oxide to the number of moles of the total sodium oxide+lithium oxide (Na₂O+Li₂O) is greater than 65%; and
ii. a total content of oxides of alkali-metal or alkaline-earth ions other than sodium and lithium below 5% relative to the total weight of the zeolitic adsorbent.

15. The process for separating xylenes according to one of claims 13 or 14, wherein the process is carried out at a temperature from 120° C. to 180° C. and wherein the water content in the hydrocarbon effluents is adjusted between 0 ppm and 80 ppm, by adding water to the feed comprising the cuts of isomers of aromatic hydrocarbons containing 8 carbon atoms and/or to the desorbent.

16. The process for separating xylenes according to one of claims 13 to 15, wherein the desorbent is selected from toluene and indane.
